# EUROPEAN PATENT APPLICATION

(11) **EP 1 302 206 A1**
(43) Date of publication of application: **16.04.2003**
(21) Application number: 01203846.9
(22) Date of filing: 11.10.2001
(51) Int. Cl.: A61K 31/739, A61K 38/39, A61K 38/16, G01N 33/50, C12N 15/00, C07K 14/00, A61K 48/00, A61P 9/10, A61P 19/00, A61K 31/715

(54) **Methods and means for the use of the adventitial toll-like receptor**

(71) Applicant: Universitair Medisch Centrum Utrecht, 3584 CX Utrecht (NL); Universiteit Utrecht, 3584 CS Utrecht (NL)
(72) Inventor: De Kleijn, Dominicus Paschalis Victor, 3962 ET Wijk bij Duurstede (NL); Pasterkamp, Gerard, 3711 CB Austerlitz (NL)
(74) Representative: Prins, Adrianus Willem

(57) **Abstract**

The present invention relates to the field of molecular biology and medicine. It particularly relates to methods and means for interfering with the formation of a neointima/scar and/or a plaque. The invention provides a method for interfering with the formation of a neointima/scar and/or a plaque in a blood vessel comprising providing a ligand capable of modulating Toll-like receptor activity of adventitial cells.

## Description

The present invention relates to the field of molecular biology and medicine. It particularly relates to methods and means for interfering with the formation of a neointima/scar and/or a plaque in a bloodvessel.

Increasing evidence exist that *C. pneumoniae* and other infectious agents play a role in atherosclerotic disease. An association between microorganisms and atherosclerosis has first been demonstrated in seroepidemiological studies [1]. In addition, *C. pneumoniae* has been detected in human atherosclerotic lesions [2]. However, the mechanism of atherogenesis by infectious organisms remains to be elucidated.

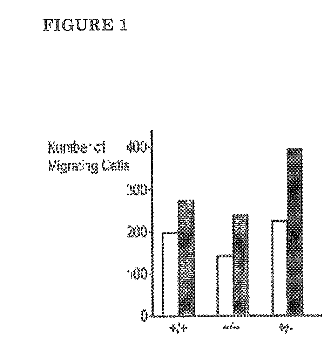

Previously, we demonstrated that the intracellular living bacterium *C. pneumoniae* is frequently present in the adventitia of atherosclerotic coronary arteries [3]. Moreover, presence of *C. pneumoniae* in the adventitia was associated with the amount and severity of atherosclerotic disease [4]. This suggested a role for adventitial cells in the development of atherosclerosis. A recent study [5] showed that adventitial fibroblasts migrate into the neointima after endothelial injury. In addition, we demonstrated [6] that migration of mouse embryonic fibroblasts was stimulated by lipopolysaccharides (LPS; Figure 1). Furthermore, adventitial application of bacterial LPS seemed to stimulate neointima formation, which was also confirmed by others [7] who showed that repetitive adventitial application of LPS or *C. pneumoniae* increased plaque formation in atherosclerotic rabbits.

Although involvement of adventitial cells in neointima/scar and/or plaque formation is evident, the biochemical machinery pathway, its components and its regulation are still unknown.

The present invention now identifies Tlr4 receptor activity as an important regulator activity in neointima/scar and/or plaque formation and subsequent atherogenesis.

In the immune system, cells are activated by bacterial wall components via, the recently characterized, Toll-like receptor 2 and 4 (Tlr2 and 4). These receptors are members of the Toll-like receptor family and are specific for bacterial wall components of gram-positive (Peptidoglycan, PG) and gram-negative bacteria (LPS), respectively. This receptor family is characterized in several cell types of the immune system, like lymphocytes and macrophages (see 8 for review). Expression of these receptors in adventitial fibroblasts has not yet been described.

Using immunochemistry on human atherosclerotic coronary arteries and primary human adventitial fibroblasts together with Western blotting and RT-PCR, we showed that Tlr4 is present in human adventitial fibroblasts (Figure 2 and Figure 3). RNAse protection and a proliferation assay showed that these fibroblasts could be activated with subsequent production of large amounts of cytokines and chemokines, which increased proliferation and/or migration of arterial smooth muscle cells. In a Tlr4 knockout mouse no intima formation was found after carotid ligation in contrast to a wildtype mouse. To see if the Tlr4 receptor was active in human primary fibroblasts, we stimulated these fibroblast with LPS and measured a large increase of mRNA levels of different cytokines and chemokines like Il-1, Il-6, MCP-1 and RANTES (Figure 4) and activation of the transcription factor NfκB (Figure 5). This revealed that adventitial fibroblasts are activated via Tlr4. This release of chemokines and cytokines by activated fibroblasts induces proliferation of smooth muscle cells (Figure 6) which then together with the adventitial fibroblasts are the cell types forming the neointima. A knock out mice study showed already that Il-1 is involved in neointima formation [14].

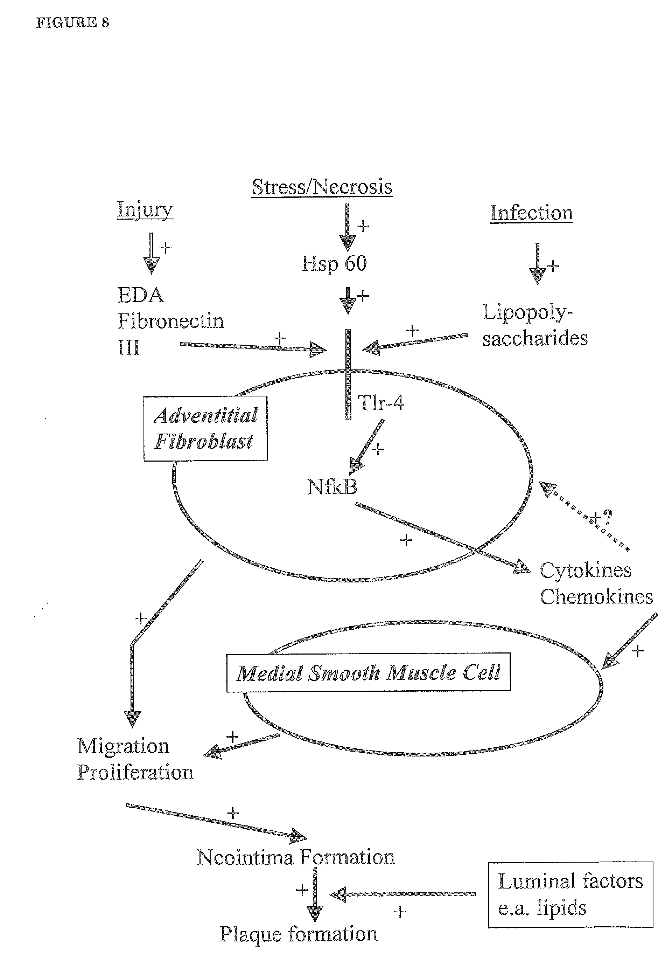

These experiments reveal a new pathway (e.g. as depicted in Figure 8) of neointima formation and subsequently atherogenesis. This pathway has its roots in the adventitia and not in the lumen or endothelium as is generally thought. Disruption of the roots of this pathway will block initiation of neointima formation. These findings clarify a potential role of Toll-like receptor activity of adventitial cells in the formation of a neointima/scar and/or a plaque.

Thus, in one embodiment the invention provides a method for interfering with the formation of a neointima/scar and/or a plaque in a blood vessel comprising providing a ligand capable of modulating Toll-like receptor activity of adventitial cells. Arteriosclerosis is a general term for a condition characterized by thickening, hardening, and loss of elasticity of the walls of the blood vessels. These changes are frequently accompanied by accumulations inside the vessel walls of lipids, e.g., cholesterol; this condition is frequently referred to as atherosclerosis. Initially lesions are formed on the arterial walls, which result in blistering and the accumulation of low-density cholesterol.
This produces higher blood pressure, which facilitates the embedding of cholesterol and calcium in the vessel walls. The fatty material accumulates calcium and produces hard plaques, thus hardening the walls of the vessels. As the vessel walls thicken, the passageways through the vessels narrow, decreasing the blood supply to the affected region. Constriction of the coronary arteries may affect the heart. If the leg vessels are affected, there may be pain with walking and an onset of gangrene. When there is total clotting of a vessel (thrombosis) the result may be a heart attack (if it occurs in the coronary arteries) or stroke (if in cerebral arteries).

One of the steps in the process of atherosclerosis is the formation of a neointima/scar and/or the formation of a plaque. Formation of a neointima/scar (in a blood vessel) typically involves the formation of (a) cell layer(s) within the lumen of the blood vessel on the luminal side of the internal elastic membrane. A plaque generally comprises a mass of fatty and cellular material that forms in and beneath the inner lining of blood vessel walls.

A blood vessel can comprise any of the vessels, such as arteries, veins, or capillaries, through which the blood circulates. The walls of a blood vessel consist of 3 layers: a tough collagen rich outer coat (the adventitia), a layer of muscular tissue (the media) and an inner layer that may encompass the initima or atherosclerotic plaques. The media and adventitia are separated by an external elastic membrane. The intima and media are separated by the internal elastic membrane. As disclosed within the experimental part, adventitial fibroblasts (which are part of the external covering of a blood vessel) of the tough outer coat, generally considered to be support cells, are immunologically active and Toll-like receptors present on these adventitial fibroblasts are an essential receptor for development of neointima scar and/or plaque formation and subsequent atherosclerosis.

A ligand of the Toll-like receptor is herein defined as a substance capable of modulating the Toll-like receptor activity and can either be an enhancer (agonist) or an inhibitor (antagonist) of Toll-like receptor activity. Examples of known enhancers of Toll-like receptor activity are lipopolysaccharides, the extra domain of fibronectin and Hsp60.

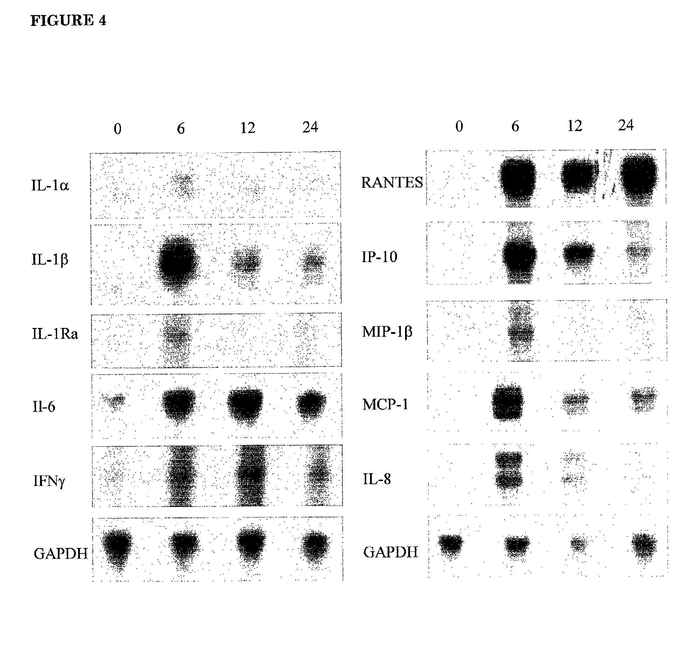

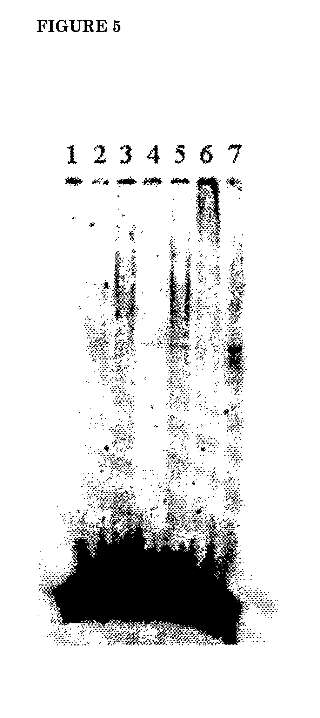

Toll-like receptor activity is herein defined as the activity brought on by the binding of a ligand to the Toll-like receptor. As disclosed herein within the experimental part, binding of lipopolysaccharides (LPS, an enhancer of Toll-like receptor activity) to the Toll-like receptor of the adventitial cells lead to an increase in mRNA levels encoding IFNγ, Il-1α, Il-1β, Il-6, Il-8, IP-10, MCP-1, MIP-1β, RANTES and the receptor Il-1Ra (Figure 4). Since activation of cytokine and chemokine production in immunological cells occurs via the transcription factor NfκB, activation of NfκB after LPS stimulation of human adventitial fibroblasts was studied using an Electrophoric Mobilty Shift Assay (EMSA, Figure 5). This revealed that NfκB was indeed translocated to the nucleus suggesting that NfκB was activated in adventitial fibroblasts after stimulation with 100 ng/ml LPS. Supershift, cold probes and non-specific probes showed that the shift was specific for NfκB. Cellular responses, like the above described increase in chemokine and/or cytokine mRNA levels, result for example in the migration and/or proliferation of blood vessel cells like medial smooth muscle cells. Therefore, the invention provides a method for interfering with the formation of a neointima/scar and/or a plaque in a blood vessel comprising providing a ligand capable of modulating Toll-like receptor activity of adventitial cells, whereby proliferation and/or migration of cells is decreased by providing a ligand capable of inhibiting Toll-like receptor activity of adventitial cells or whereby proliferation and/or migration of cells is increased by providing a ligand capable of enhancing Toll-like receptor activity of adventitial cells.

It is clear to a person skilled in the art that the present invention not only provides a method for interfering with the formation of a scar in a blood vessel by comprising providing a ligand capable of modulating Toll-like receptor activity of adventitial cells, but that also other scar tissue, for example, scar formation on the skin (as a result of operations or more specifically as a result of plastic surgery) can be modulated according to a method of the invention. In case of surgery, the Toll-like receptor, present on blood vessels within or surrounding the operated area, is activated which results in the migration of fibroblasts and eventually in the formation of skin scar tissue.

In a preferred embodiment the invention provides a method for interfering with the formation of a neointima/scar and/or a plaque in a blood vessel according to the invention wherein said Toll-like receptor activity is Toll-like receptor 4 (Tlr4) activity. The Toll-like receptor family is characterized in several cell types of the immune system, like lymphocytes and macrophages (see reference 8 for review). It was not until the present invention that expression of the Toll-like 4 receptor on adventitial fibroblasts has been described and even more important that activation of the Toll-like 4 receptor leads to an increase in mRNA levels of different cytokines and chemokines which in turn lead to proliferation and migration of blood vessel cells (for example, fibroblasts and/or smooth muscle cells). The adventitial fibroblasts, which were generally considered to be support cells, are immunological active and are an essential receptor for development of neointima/scar formation and/or plaque formation and subsequent atherosclerosis.

In another embodiment the invention provides a method for reducing the formation of a neointima/scar and/or a plaque in a blood vessel after stenting or after angioplasty or after heart transplantation or after by pass surgery or after arterio-venous shunting, comprising providing a ligand capable of inhibiting Toll-like receptor activity (preferably Toll-like receptor 4 activity) of adventitial cells.

Stenting typically involves intra-luminal placement of a metal or a biodegradable device that, after being distended by balloon inflation, will keep its distended diameter and hence will prevent elastic recoil of the arterial wall.

Angioplasty is a general term for any surgical repair of a blood vessel, especially balloon angioplasty or percutaneous transluminal coronary angioplasty, a treatment of coronary artery disease. In balloon angioplasty a balloon-tipped catheter is inserted through the skin into a blood vessel and manoeuvred to the clogged portion of the artery. There it is threaded into the blockage and inflated, compressing the plaque against the arterial walls.

By pass surgery is the surgical revascularization of the heart, using healthy blood vessels of the patient, performed to circumvent obstructed coronary vessels and improve blood flow.

Arterio-venous shunting is a process in which typically a direct connection between an artery and a vein is created. This process is for example used to provide a kidney patient with an easy accessible entrancepoint for the necessary extra corporeal dialysis.

A frequent postoperative problem in all these treatments is reclogging (restenosis) of the treated area and/or the formation of a neotinitima/scar and/or a plaque. By providing a ligand capable of inhibiting Toll-like receptor activity (preferably Toll-like receptor 4 activity) of adventitial cells the formation of a neointima/scar and/or a plaque in a blood vessel after stenting or after angioplasty or after heart transplantation or after by pass surgery or after arterio-venous shunting is diminished or more preferably completely inhibited.

In yet another embodiment the invention provides a method for reducing the formation of a neointima/scar and/or a plaque in a blood vessel after infection, more preferably after a bacterial infection, comprising providing a ligand capable of inhibiting Toll-like receptor activity (preferably Toll-like receptor 4 activity) of adventitial cells. Natural (bacterial) infection or a (bacterial) infection as a negative side effect after, for example, a treatment like stenting, angioplasty, heart transplantation, by pass surgery or arterio-shunting results in the production of LPS which in turn activates the Toll-like receptor on adventitial cells and thereby stimulates neointima/scar formation and/or plaque formation and subsequent atherosclerosis. By providing a ligand capable of inhibiting Toll-like receptor activity (preferably Toll-like 4 receptor activity) of adventitial cells said neointima/scar formation and/or plaque formation and subsequent atherosclerosis is diminished or more preferably completely inhibited.

Mammalian cells require oxygen and nutrients for their survival and are therefore located within 100 to 200 µm of blood vessels. For multicellular organisms to grow beyond this size, they must recruit new blood vessels by vasculogenesis and angiogenesis. This process is regulated by a balance between pro- and antiangiogenic molecules, and is derailed in various diseases, especially cancer. Without blood vessels, tumors cannot grow beyond a critical size or metastasize to another organ. In the later phases in the process of angiogenesis proliferation and/or migration of cells play an important role.

By providing a ligand capable of modulating Toll-like receptor activity (preferably Toll-like receptor 4 activity) of adventitial cells, cell migration is modulated and thus especially the late phases in the process of angiogenesis (being e.g. fibroblast migration) are modulated. Furthermore the invention provides a method for modulating tumor growth (primary or metastatic) by interfering with the formation of a neointima/scar and/or a plaque in a blood vessel comprising providing a ligand capable of modulating Toll-like receptor activity of adventitial cells. By stimulation of the Toll-like receptor (preferably Toll-like receptor 4) more neointima are formed which eventually block a blood vessel and thereby prevent the flow of blood to a (growing or forming of) tumor.

The invention further provides a method for modulating effects of rheumatoid arthritis by interfering with the formation of a neointima/scar and/or bone and/or cartilage degrading cells in a joint comprising providing a ligand capable of modulating Toll-like receptor activity (preferably Toll-like receptor 4 activity) of adventitial cells, whereby proliferation and/or migration of cells is modulated. One aspect of rheumatoid arthritis is the influx of fibroblasts into the synovium and the stimulation of these fibroblasts to produce inflammatory mediators, which eventually leads to joint destruction. By providing a ligand capable of inhibiting Toll-like receptor activity of adventitial cells the migration of fibroblasts is inhibited and therefore the effects of rheumatoid arthritis are inhibited (or more preferably completely diminished). Furthermore angiogenesis is also believed to be involved in rheumatoid arthritis. The blood vessels in the synovial lining of the joints undergo angiogenesis. By providing modulating Toll-like receptor activity (preferably Toll-like receptor 4 activity) the effects of rheumatoid arthritis are modulated. Migration and accumulation of fibroblast-like cells play an important role in rheumatoid arthritis because these are important producers of the bone and cartilage degrading metalloproteases. Inhibition of the Toll-like receptor activity (preferably Toll-like receptor 4 activity) reduces (or preferably completely inhibits) the migration of the fibroblast-like cells and reduces (or preferably completely inhibits) the effects observed in rheumatoid arthritis.

Preferably said Toll-like receptor activity (more preferably Toll-like receptor 4 activity) is modulated by a specific binding agent (or ligand, the terms are used interchangeably herein) for Toll-like receptor and even more preferably said specific binding agent is a protein and/or a functional derivative and/or a functional fragment thereof. It is clear to a person skilled in the art that only the essential part or parts of such a specific binding agent are required for the invention. Therefore, a functional derivative and/or functional fragment thereof is herein defined as a derivative and/or fragment capable of modulating Toll-like receptor activity possibly in different amounts. Another way to modulate the Toll-like receptor activity (preferably Toll-like receptor 4 activity) is by modulating the availability of ligand-co-binding compounds. For example, the ligand LPS binds to the Toll-like receptor (preferably Toll-like receptor 4) through the action of CD14. By modulating the availability of CD14, for example by providing an anti-CD14 antibody which captures the CD14, the binding of LPS is reduced or more preferably completely inhibited.

In yet another embodiment the invention provides a method for identifying a ligand of Toll-like receptor activity (preferably Toll-like receptor 4 activity) of adventitial fibroblasts by screening a compound library comprising
- culturing adventitial fibroblasts comprising a Toll-like receptor
- inducing said Toll-like receptor
- providing a testcompound to said fibroblasts
- testing medium surrounding said fibroblasts for cytokine and/or chemokine levels
Since all the steps can be performed in ELISA plates, this assay is very easy to perform by autoanalysers, yielding a high throughput. Such an assay to screen a compound library for Tlr4 ligands comprises the next steps:
- Human adventitial fibroblasts (also commercially available) are used as the bioassay and cultured in DMEM with Glutamax (Gibco BRL), Penicillin 100 U/ml, Streptomycin 100 µg/ml and 10% fetal bovine serum.
- Equal amounts of human adventitial fibroblasts will be cultured in 96 wells of an ELISA plate.
- After attachment O/N (overnight), medium is changed and replaced by new medium or medium containing ∼10 ng/ml LPS or ∼10 µg/ml Hsp60 or 30-300 nM EDA fibronectin.
- The compound to be tested is added (possibly in different concentrations) to each of the different media.
- This can be extended to incubate first with medium plus compound followed by compound plus activator.
- After 6-8 h at 37°C/5% CO₂ medium is harvested and stored at -20°C
- Or medium is used immediately in ELISA measuring release of specific cytokine or chemokine; Il-1, IL-6, Il-8, MCP and RANTES are all good candidates.
- Amount of cyto/chemokine is compared between incubation with and without the compound.
- Only DMEM is used to subtract for the cyto/chemokines present in FCS (background control).

In yet another embodiment the invention provides an isolated, recombinant or synthetic Toll-like receptor activity (preferably Toll-like receptor 4 activity) inhibitor obtainable according to a method of the invention. In a preferred embodiment the invention provides an isolated, recombinant or synthetic Toll-like receptor activity inhibitor according to the invention wherein said inhibitor is a protein (proteinaceous inhibitor of Toll-like receptor activity) and/or a functional derivative and/or a functional fragment thereof. A functional derivative and/or functional fragment thereof is herein defined as a derivative and/or fragment capable of modulating Toll-like receptor activity possibly in different amounts.

In yet another embodiment the invention provides a nucleic acid encoding a proteinaceous inhibitor of Toll-like receptor activity according to the invention. Furthermore the invention provides a vector comprising a nucleic acid according to the invention. In another embodiment the invention provides a gene delivery vehicle comprising a vector according to the invention.

In yet another embodiment the invention provides a pharmaceutical composition comprising a Toll-like receptor activity inhibitor, a nucleic acid, a vector or a gene delivery vehicle according to the invention. Preferably such a pharmaceutical is used for decreasing the formation of a neointima/scar and/or a plaque in a blood vessel.

In a more preferred embodiment the invention provides use of a ligand capable of modulating Toll-like receptor activity of adventitial cells for the preparation of a pharmaceutical composition for interfering with the formation of a neointima/scar and/or a plaque in a blood vessel. Even more preferably such a pharmaceutical composition is used for the reduction (or more preferably completely diminishing) of (cardio) vascular damage after stenting, balloon angioplasty, heart transplantation, by pass surgery, arterio-venous shunting or infection.

In yet another embodiment the invention provides a method for reducing the amount of bone and/or cartilage degrading cells in rheumatoid arthritis comprising a ligand capable of inhibiting Toll-like receptor activity.

The invention will be explained in more detail in the following detailed description, which is not limiting the invention.

### EXPERIMENTAL PART

### Animals

Female wild type C3H/HeN mice with a normal Tlr4 receptor and C3H/HeJ with a non-functional Tlr4 receptor were anesthetized using 0.05 ml/10 g body weight of a cocktail (1 part Hypnorm, 1 part of Midazolam, 2 parts of distilled water). The right common carotid artery was ligated as described by Kumar & Lindner (17). Animals were terminated at 28 days after ligation. Before harvesting, the arteries were perfused via the left ventricle for 3 min. with phosphate buffered saline (PBS) plus 10⁻⁴ M sodium nitroprusside followed by a 3 min. perfusion with 4% paraformaldehyde in PBS plus 10⁻⁴ M sodium nitroprusside at 99 ml/h. After harvesting, arteries were fixed in 4% paraformaldehyde in PBS for several days before embedded in paraffin. Eight or more serial sections (6 µm), spanning most of the vessel segment, were cut and stained with elastin von Gieson and hematoxilin-eosin for morphometry. All sections were analyzed for morphometry using the SIS analysis software package.

The investigation conformed to the Guide for the Care and Use of Laboratory Animals (NIH publication No.85-23, 1985) and was approved by the ethical committee on animal experiments of the Faculty of Medicine, Utrecht University and University of Singapore.

### Mouse primary fibroblasts

Mouse primary fibroblasts were prepared as previously described. Briefly, 12.5 dpc pregnant Balb/c mice were sacrificed and the embryos were removed. The embryos were eviscerated and mechanically dispersed using a tissue sieve and successive passages through 18G, 21G, 23 and 24 G needles. The cells were plated at a density of 2 embryos per plate (diameter 10 cm) grown in DMEM with 15% FCS and β-mercaptoethanol (7.2 µl/l).
Mouse primary fibroblasts were cultured in DMEM (GibcoBRL) with MEM non-essential amino acids 1X (GibcoBRL), 3.6 µl/500 ml β-Mercaptoethanol, Penicillin 100 U/ml, Streptomycin 100 µg/ml and 20% fetal bovine serum. Migration was studied using transwell culture chambers (Costar 3422) with approximately 20,000 cells per chamber. Migration was performed for 20h according to manufacturer's protocol.

### Human adventitial fibroblasts

A small part of the human aorta was dissected from a human donor or recipient heart. The adventitial layer was stripped from the aorta, rinsed several times in PBS, cut in small pieces and incubated O/N at 37°C in PBS and collagenase (1/20, La Roche).

Next, the adventitial tissue is resuspended in a 1 ml pipetpoint and transferred in a 15 ml tube. Cells are collected after 5 min centrifugation at 1000 rpm. The collagenase solution is removed and cells are resuspended in culture medium (DMEM with Glutamax, Penicillin 100 U/ml, Streptomycin 100 µg/ml and 10% fetal bovine serum) and transferred to a culture flask. Cells were allowed to attach for 6 hours. Cells not attached were removed at that time.

### Extraction of RNA and protein

After collection, a small part of the human coronary artery was dissected and fixed for 2 h in 4% paraformaldehyde at room temperature (RT), followed by an overnight (O/N) incubation at 4°C in 15% sucrose in PBS, and embedded in Tissue Tec (Miles Inc.) and stored at -80°C until use for in situ hybridization or immunohistochemistry. The remaining artery was frozen and ground with a pestle and mortar in liquid nitrogen until a fine powder was obtained and used for Tripure (Roche) isolation of RNA and protein.

Extraction and purification of total RNA and protein from cultured cells was performed according to manufacturer's protocol.

### RNAse protection assay

Total RNA (2 µg) was used in an RNAse protection assay (Ambion). cRNA probes were generated with 1 ug template (HCK-2, HCK-3, HCK-5;Ambion). Probe synthesis and protection assay were performed according manufacturers protocols.

Protected fragments were separated on a 4% urea acryl amide gel. After running, the gel was exposed to a Biomax film (Amersham). Protected fragments were quantified and compared to the housekeeping gene GAPDH using the Gel Doc 1000 system (Biorad).

### Western blotting

For Western blotting, protein samples were separated on a SDS polyacrylamide gel (8%). After electrophoresis, the gel was blotted to Hybond-C (Amersham) in blotting buffer (3.03 g/L Tris 14.4 g/L Glycine, 20% methanol). After blotting, the gel was blocked O/N in PBS, 0.1% Tween 20 (PBST) and 5% non-fat dry milk (Protifar, Nutricia). Incubation with the first antibody (rabbit anti-human Tlr-4 1/1000) occurred in PBST plus 5% Protifar for 1 hr at RT followed by washing (3x 5 min.) with PBST.

Incubation was continued using goat anti-rabbit biotin (1/1000, DAKO) for 1 hr at RT in PBST plus 5% Protifar followed by washing (3x) with PBST. After this, incubation with streptavidin/peroxidase (1/2000, Southern Biotechnology) occurred at RT for 1 hr, in PBST plus 5% Protifar. The blot was washed two times with PBST. Bands were visualized using the ECL kit (Amersham)

### Immunohistochemistry

Arterial segments were cut into 8 µm sections and transferred to Superfrost plus slides (Menzel Glazer) and stored at -80°C until use. Sections were fixed for 10 minutes in acetone containing 0.03 % H₂O₂ to block endogenous peroxidase. Next, sections were incubated with 10 % normal goat serum for 30 minutes (RT) and in addition, incubated with rabbit anti human Tlr4 1/1000 overnight at 4°C in PBS/BSA 0.1 %. After overnight incubation the sections were rinsed in PBS (three times for 5 minutes) and incubated with 1 µg/ml goat anti-rabbit HRPO PAb (Dakopatts) in PBS/BSA 1% containing 1% normal rabbit serum (1 hr, RT). Next, the sections were rinsed in PBS (three times for 5 minutes).

To visualize the horseradish peroxidase, the sections were treated with a sodium acetate buffer containing 0.4 mg/ml 3-amino 9-ethylcarbazole substrate for 10 minutes.

For staining of human primary fibroblasts, cells were grown on Superfrost plus slides, fixed with ice-cold 4% formaldehyde for 20 min. and subsequently treated with 1% Triton X100 in PBS. Next, sections were incubated with 10 % normal goat serum for 30 minutes (RT) and in addition, incubated overnight with rabbit anti human Tlr4 1/1000 or mouse anti human Vimentin 1/1000 at 4°C in PBS/BSA 0.1 %. Tlr4 staining was visualized using biotin labeled horse anti mouse and subsequently with Texas Red labeled streptavidine. Vimentin staining was visualized using biotin labeled goat anti rabbit and FITC labeled streptavidine.

### RT-PCR and sequence analysis

Total RNA (500 ng) was converted to cDNA using the Ready to GoYou Prime system (Pharmacia) with 1 µl (200 ng) hexanucleotide according to the instructions of the manufacturer. After cDNA synthesis, the sample was diluted with DEPC treated water to 540 µl. For PCR amplification of human Tlr-4 cDNA, reactions (25 µl) contained 200 µM dNTP, 1 x PCR reaction buffer (Pharmacia), 2.5 U Taq DNA polymerase (Pharmacia), 1 µM of each primer (5' TCAGCTCTGCCTCTACTAC 3'and 5' ACACCACAACAATCACCTTTC 3') and 20 µl diluted cDNA. A typical PCR started with a 2 min incubation at 94°C, 30 sec. at 94°C, 30 sec. at 50°C and 30 sec. at 72°C followed by an extension of 7 min. at 72°C for 30 cycles. Ten µl of each reaction was electroforesed through 8 % polyacrylamide gels. After running, the gel was stained with EtBr and visualized using the Gel Doc 1000 system.

The identity of the amplified human Tlr-4 cDNA was confirmed by subcloning the amplified cDNA into pGEM-T Easy (Promega) and then sequencing the inserts (Amersham, Sequenase 2.0).

### Cell proliferation assay

Human arterial smooth muscle cells (ATCC: CRL 1999, passage 22-26) were used. Cells were grown in Ham's F12 medium with 2 mM L-glutamine, 1.5 g/L sodium bicarbonate, 10 mM HEPES, 10 mM TES, 50 µg/ml ascorbic acid, 10 µg/ml insulin, 10 µg/ml transferrin, 10 ng/ml sodium selenite, 30 µg/ml endothelial cell growth supplement and 10% fetal bovine serum. During experiments, growth was arrested by using only 0.2 % fetal bovine serum for 24 h. After this cells were cultured O/N in 0.5% fetal bovine serum. Proliferation was determined by adding BrdU and the cell proliferation ELISA kit (Roche) according to manufacturers protocol.

### Statistical analysis

Statistical analysis of the results was performed by the students t-test. Data are presented as mean ± standard deviation. Differences were considered as statistically significant for p-values less then 0.05.

### RESULTS

### Identification of Tlr4 in human adventitial fibroblast

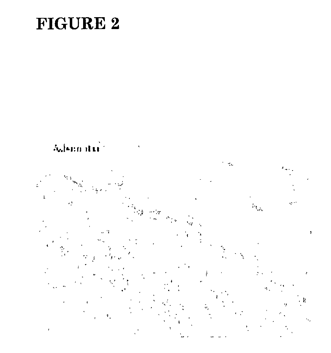

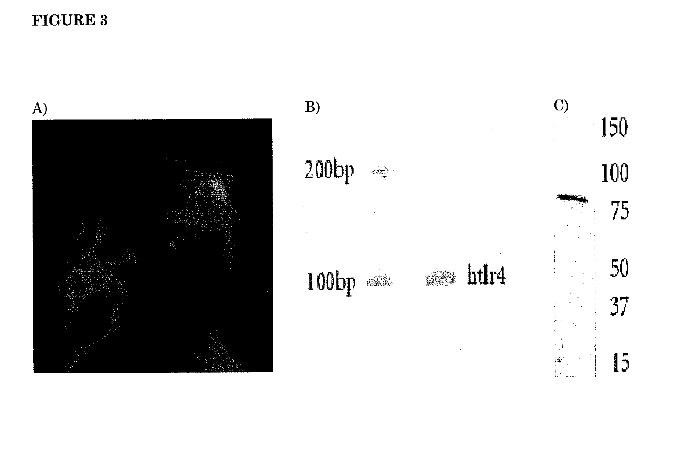

Human atherosclerotic coronary arteries were used to localize Tlr4 protein (Figure 2). Tlr4 was found in the atherosclerotic plaque but a large number of positive cells were found in the adventitia. Due to their morphology, we assumed that adventitial fibroblasts were positively stained for Tlr4. To investigate this, we stripped the adventitial layer of human aortas and isolated the primary adventitial fibroblast. Immunochemistry (Figure 3a) not only identified the isolated cells as fibroblasts using a vimentin staining but also showed that in the same cells Tlr4 was present. RT-PCR (Figure 3b) showed that the expected 100 bp PCR product was present in the adventitial fibroblasts encoding a part of Tlr4 (results not shown). Western blotting on protein extracted from adventitial fibroblasts revealed a band of the expected size (Figure 3c). These experiments identify the presence of Tlr4 in human adventitial fibroblast.

### Activation of Tlr4 in human adventitial fibroblasts

The presence of Tlr4 does not provide evidence for Tlr4 activation and function in adventitial fibroblasts. In the presence of LPS, immunological cells like monocytes produce large amounts of cytokines and chemokines [8]. Therefore, we stimulated human adventitial fibroblast with 100 ng/ml LPS and measured in time mRNA levels of different cytokines and chemokines (Figure 4). This revealed a large increase in mRNA levels encoding IFNγ, Il-1α, I1-1β, I1-6, I1-8, IP-10, MCP-1, MIP-1β, RANTES and the receptor Il-1Ra (Figure 4). Especially the increase of MCP-1 points to initiation of monocyte homing by the fibroblast. Since activation of cytokine and chemokine production in immunological cells occurs via the transcription factor NfκB, we studied NfκB activation after LPS stimulation of human adventitial fibroblasts using an Electrophoric Mobilty Shift Assay (EMSA, Figure 5). This revealed that NfκB was indeed translocated to the nucleus suggesting that NfκB was activated in adventitial fibroblasts after stimulation with 100 ng/ml LPS. Supershift, cold probes and non-specific probes showed that the shift was specific for NfκB.

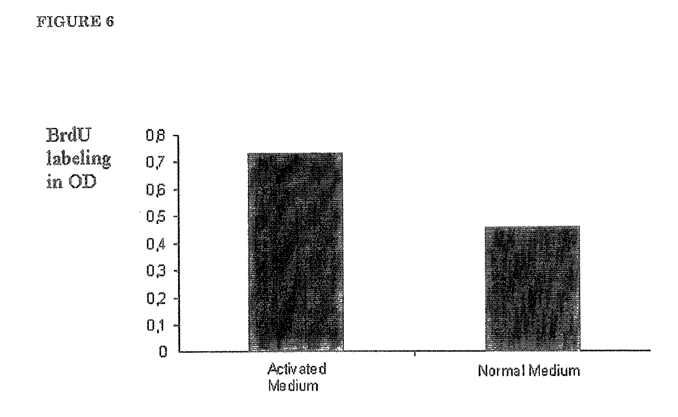

Having demonstrated that Tlr4 can be activated in human adventitial fibroblasts, we hypothesized that these fibroblasts can initiate cell migration and proliferation of medial smooth muscle cells. Therefore, we incubated arterial smooth muscle cells with medium derived from human adventitial fibroblast with or without being stimulated with LPS and measured proliferation of the smooth muscle cells using incorporation of BrdU. This revealed that the release of chemo and cytokines by LPS-activated fibroblasts can induce proliferation of smooth muscle cells (Figure 6). This suggests that smooth muscle cell proliferation can be initiated by adventitial fibroblast Tlr4 activation. Together with the adventitial fibroblasts these smooth muscle cells are the cell types that form the neointima. This is confirmed by a knock out mice study showing that Il-1 is involved in neointima formation [14].

### Unilateral common carotid ligation in wild type and Tlr4 knockout mice

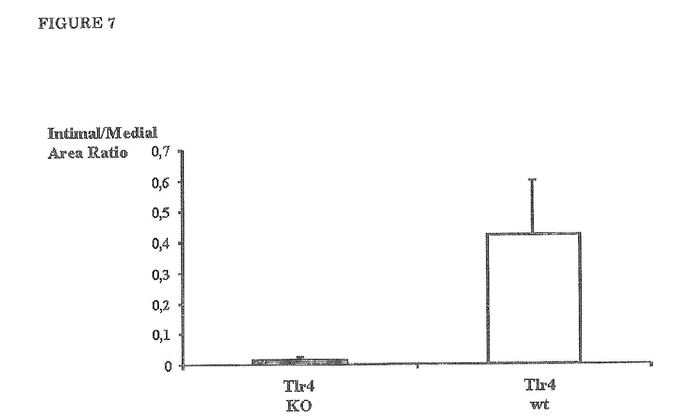

After inducing arterial injury via flow cessation in the carotid artery in wt and Tlr4 knockout mouse, we found no neointima formation in the Tlr4 KO and a large amount of neointima formation in the wt mouse (Figure 7). This shows that Tlr4 is essential in initiating neointima formation after arterial injury.

In conclusion, these experiments reveal a new pathway (Figure 8) of neointima formation and subsequently atherogenesis, which has its roots in the adventitia and not in the lumen or endothelium as, is generally considered. Interfering in the roots of this pathway will block the initiation of neointima formation after injury like balloon angioplasty, stenting, heart transplantation, by pass surgery and arterio-venous shunting but also after infection with bacterial wall components. This pathway should also be available for targeting in other functional and pathological processes in which tissue scarring with cell migration is observed, like tissue repair after injury and ischemia in other tissues, infectious diseases, rheumatoid arthritis and tumor fibrogenesis.
1. Danesh J, Collins R, Peto R. Chronic infections and coronary heart disease: is there a link? (1997) Lancet; 350:430-436.
2. Taylor-Robinson D, Thomas BJ. Chlamydia pneumoniae in arteries: the facts, their interpretation and future studies. (1998) J. Clin. Pathol.;51:793-797.
3. Vink A, Pasterkamp G, Poppen M, Schoneveld AH, De Kleijn DPV, Roholl P, Fontijn J, Plomp S, Borst C. The adventitia of atheroslerotic coronary arteries frequently contains Chlamydia Pneumoniae. Atherosclerosis, in press
4. Vink A, Pasterkamp G, Poppen M, Schoneveld AH, Roholl PJM, De Kleijn DPV, Borst C. Distribution of Chlamydia Pneumoniae in the human arterial system and its relation with the local amount of atherosclerosis within the individual. (2001) Circulation 103,1613-1617.
5. Li G, Chen SJ, Oparil S, Chen YF, Thompson JA. Direct in vivo evidence demonstrating neointimal migration of adventitial fibroblasts after balloon injury of rat carotid arteries. (2000) Circulation 101, 1362-1365.
6. De Kleijn DPV, Smeets MB, Kemmeren PPCW, Lim SK, Velema E, Schoneveld A, Pasterkamp G, Borst C. The acute phase protein haptoglobin is a cell migration factor involved in arterial restructuring. J. Exp. Med., submitted for publication.
7. Engelmann et al.,(2000) Eur. Heart. J. 21, 456
8. Anderson KV. Toll signaling pathways in the innate immune response (2000) Current opinion in Immunology, 12:13-19
9. Okamura Y, Watari M, Jerud ES, Young DW, Ishizaka ST, Rose J, Chow JC, Strauss JF 3rd. The extra domain A of fibronectin activates Toll-like receptor 4. (2001) J. Biol. Chem. 276, 10229-10233
10. George J, Wang SS, Sevcsik AM, Sanicola M, Cate RL, Koteliansky VE, Bissell DM Transforming growth factor-beta initiates wound repair in rat liver through induction of the EIIIA-fibronectin splice isoform. (2000) Am. J. Pathol. 156, 115-124.
11. Saito S, Yamaji N, Yasunaga K, Saito T, Matsumoto S, Katoh M, Kobayashi S, Masuho Y. The fibronectin extra domain A activates matrix metalloproteinase gene expression by an interleukin-1-dependent mechanism. (1999) J. Biol. Chem. 274, 30756-30763.
12. Chen W, Syldath U, Bellmann K, Burkart V, Kolb H. Human 60-kDa heat-shock protein: a danger signal to the innate immune system. (1999) J. Immunol. 162, 3212-3219.
13. Ohashi K, Burkart V, Flohe S, Kolb H. Cutting edge: heat shock protein 60 is a putative endogenous ligand of the toll-like receptor-4 complex. (2000) J. Immunol. 164, 558-561.
14. Rectenwald, J.E., Moldawer, L.L., Huber, T.S., Seeger, J.M., Ozaki, C.K. Direct evidence for cytokine involvement in neointimal hyperplasia. (2000) Circulation 102, 1697-1702
15. Kumar, A., Lindner, V. (1997). Remodeling with neointima formation in the mouse carotid artery after cessation of blood flow. Arterioscler. Thromb. Vasc. Biol. 17, 2238-2244.
16. Lardenoye JH, Delsing DJ, de Vries MR, Deckers MM, Princen HM, Havekes LM, van Hinsbergh VW, van Bockel JH, Quax PH. Accelerated atherosclerosis by placement of a perivascular cuff and a cholesterol-rich diet in ApoE*3Leiden transgenic mice. Circ. Res. 2000 87,248-53.

### DESCRIPTION OF FIGURES

Figure 1 Number of migrating mice embryonic fibroblasts (+/+ = wt, +/- = heterozygous for haptoglobin without (white bars) and with stimulation (10 ng/ml LPS, black bars); P=0.02
Figure 2 Immunohistochemistry of a human atherosclerotic coronary artery cross-section using an antibody directed against human Toll-like receptor 4. Positive staining (brown) is found in adventitia
Figure 3 Identification of human Toll-like receptor 4 (Tlr4) in human primary adventitial fibroblasts A) Double staining of human primary fibroblasts using an antibody against the fibroblast marker vimentin (red) and Tlr4 (green). B) RT-PCR specific for human Tlr4 on RNA isolated from human primary adventitial fibroblasts. The 100 bp band coded for a fragment of Tlr4 (sequence results not shown). C) Western blotting on proteins extracted from human primary adventitial fibroblasts using an antibody against human Tlr4 showed the expected 75 kD band.
Figure 4 RNase Protection Assay of different chemokine and cytokine mRNAs on RNA extracted from human primary adventitial fibroblast 0, 6, 12 and 24 h after stimulation with 10 ng/ml LPS. The housekeeping mRNA GAPDH is used as an internal control.
Figure 5 Electrophoretic Mobility Shift Assay with human adventitial fibroblasts after stimulation with LPS. Lanes: 1 negative control 2 no LPS 3 LPS 4 cold probe 5 non-specific cold probe 6 NfkB probe plus NfkB antibody (α-p65) 7 positive control.
Figure 6 Proliferation of arterial smooth muscle cells after O/N incubation with medium of adventitial fibroblasts activated with LPS or unactivated. Proliferation was measured using BrdU labeling and a BrdU ELISA.
Figure 7 The neointima formation response of the left common carotid artery from Tlr-4 KO (black bar) and wt (white bar) mice, 4 weeks after ligation.
Figure 8 A possible pathway of neointima formation and subsequent atherogenesis originating in the arterial adventitial layer.

## Claims

1. A method for interfering with the formation of a neointima/scar and/or a plaque in a blood vessel comprising providing a ligand capable of modulating Toll-like receptor activity of adventitial cells.

2. A method for interfering with the formation of a neointima/scar and/or a plaque in a blood vessel according to claim 1, whereby proliferation and/or migration of cells is decreased by providing a ligand capable of inhibiting Toll-like receptor activity of adventitial cells.

3. A method for interfering with the formation of a neointima/scar and/or a plaque in a blood vessel according to claim 1, whereby proliferation and/or migration of cells is increased by providing a ligand capable of enhancing Toll-like receptor activity of adventitial cells.

4. A method for interfering with the formation of a neointima/scar and/or a plaque in a blood vessel according to anyone of claims 1 to 3 wherein said Toll-like receptor activity is Toll-like receptor 4 activity.

5. A method for reducing the formation of a neointima/scar and/or a plaque in a blood vessel after stenting comprising providing a ligand capable of inhibiting Toll-like receptor activity of adventitial cells.

6. A method for reducing the formation of a neointima/scar and/or a plaque in a blood vessel after angioplasty comprising providing a ligand capable of inhibiting Toll-like receptor activity of adventitial cells.

7. A method for reducing the formation of a neointima/scar and/or a plaque in a blood vessel after heart transplantation comprising providing a ligand capable of inhibiting Toll-like receptor activity of adventitial cells.

8. A method for reducing the formation of a neointima/scar and/or a plaque in a blood vessel after by pass surgery comprising providing a ligand capable of inhibiting Toll-like receptor activity of adventitial cells.

9. A method for reducing the formation of a neointima/scar and/or a plaque in a blood vessel after arterio-venous shunting comprising providing a ligand capable of inhibiting Toll- like receptor activity of adventitial cells.

10. A method for reducing the formation of a neointima/scar and/or a plaque in a blood vessel after infection comprising providing a ligand capable of inhibiting Toll-like receptor activity of adventitial cells.

11. A method for reducing the formation of a neointima/scar and/or a plaque in a blood vessel according to claim 10 wherein said infection is a bacterial infection.

12. A method for modulating tumor growth by interfering with the formation of a neointima/scar and/or a plaque in a blood vessel comprising providing a ligand capable of modulating Toll-like receptor activity of adventitial cells.

13. A method for modulating effects of rheumatoid arthritis by interfering with the formation of a neointima/scar and/or bone and/or cartilage degrading cells in a joint comprising providing a ligand capable of modulating Toll-like receptor activity of adventitial cells.

14. A method according to anyone of claims 5 to 13 wherein said Toll-like receptor activity is Toll-like receptor 4 activity.

15. A method according to anyone of claims 5 to 14 whereby Toll-like receptor activity is inhibited by a specific binding agent for Toll-like receptor.

16. A method according to claim 15 wherein said specific binding agent is a protein and/or a functional derivative and/or a functional fragment thereof.

17. A method for identifying a ligand of Toll-like receptor activity of adventitial fibroblasts by screening a compound library comprising
- culturing adventitial fibroblasts comprising a Toll-like receptor
- inducing said Toll-like receptor
- providing a testcompound to said fibroblasts
- testing medium surrounding said fibroblasts for cytokine and/or chemokine levels

18. An isolated, recombinant or synthetic Toll-like receptor activity inhibitor obtainable according to the method of claim 17.

19. An isolated, recombinant or synthetic Toll-like receptor activity inhibitor according to claim 18 wherein said inhibitor is a protein and/or a functional derivative and/or a functional fragment thereof.

20. A nucleic acid encoding a Toll-like receptor activity inhibitor according to claim 19.

21. A vector comprising a nucleic acid according to claim 20.

22. A gene delivery vehicle comprising a vector according to claim 21.

23. A pharmaceutical composition comprising a Toll-like receptor activity inhibitor according to claim 18 or 19, a nucleic acid according to claim 20, a vector according to claim 21 or a gene delivery vehicle according to claim 22.

24. A pharmaceutical composition according to claim 23 for decreasing the formation of a neointima/scar and/or a plaque in a blood vessel.

25. Use of a ligand capable of modulating Toll-like receptor activity of adventitial cells for the preparation of a pharmaceutical composition for interfering with the formation of a neointima/scar and/or a plaque in a blood vessel.

26. Use according to claim 25 for the reduction of (cardio) vascular damage after stenting, balloon angioplasty, heart transplantation, by pass surgery, arterio-venous shunting or infection.

27. A method for reducing the amount of bone and/or cartilage degrading cells in rheumatoid arthritis comprising a ligand capable of inhibiting Toll-like receptor activity.
